# EUROPEAN PATENT APPLICATION

(11) **EP 2 835 144 A1**
(43) Date of publication of application: **11.02.2015**
(21) Application number: 14174633.9
(22) Date of filing: 27.06.2014
(51) Int. Cl.: A61M 5/20

(54) **Injection device with stopper assembly**

(30) Priority: 28.06.2013 CH 11882013
(71) Applicant: juvaplus SA, 2000 Neuchâtel (CH)
(72) Inventor: Besuchet, Romain, 1400 Yverdon les Bains (CH); Perriard, Yves, 2000 Neuchâtel (CH); Legrand, Bernard-Pierre, 1231 Conches (CH)
(74) Representative: P&TS SA (AG, Ltd.)

(57) **Abstract**

An injection device for injecting injection material, comprising :
an elastic deformable element (3) able to exert a pushing force on a piston (20) of a syringe (2) so as to eject a quantity of injection material out of the syringe (2);
a stopper (4) for retaining the extension of said spring (3) when the stopper is in a first position (A) and for releasing the extension of said elastic deformable element when the stopper is in a second position (B);
an electric actuator (5) for moving the stopper (4) from its first position to its second position or from its second position to its first position.

## Description

### Technical field

The present invention pertains to an injection device, in particular an injection device for aesthetic or surgical procedures, for administering a liquid or viscous material beneath the skin of a person by means of a syringe. The injection device accommodates a syringe comprising at least a syringe barrel for the injection material, a syringe needle and a piston located in the syringe and drivable by a plunger for ejecting a quantity of injection material out of the syringe.

### State of the art

A significant part of efforts in aesthetic medicine is directed to so called face injections. Various types of injection materials are commonly used in aesthetic medicine, including filler materials as well as botulinum toxin, often called by the brand name "Botox". Fillers are injected to fill the underneath of a wrinkle that pre-exists on the patient's skin. Botulinum toxin de-stimulates muscles with the result that the person is unable to use a certain muscle group.

Botulinum toxin shows a low viscosity, like water. Only small amounts have to be administered in order to de-stimulate a patient's specific muscle. On the other hand, dermal fillers such as hyaluronic acid are very viscous liquids. Face filling involves filling the skin with a "filler" material in order to create volume underneath the skin.

The manner in which fillers and botulinum toxin are injected is different due to the volume injected per patient, the viscosity of the fluids, and the way the fluid is deposited underneath the skin. The present invention is focused on providing a device for injection of both dermal fillers and botulinum toxin or similar products.

The injection of dermal fillers requires a significant force to expel the high viscosity fluid out of the syringe. It is however difficult for the physician to control precisely the injection point while simultaneously applying a large force on the piston of the syringe. Therefore, a syringe that does not require a large force to be exerted by the physician when he is making the injection would be beneficial.

An injection device that requires less force would be beneficial not only for injecting high viscosity fluids such as dermal fillers, but also for injecting less viscous fluids such as botulinum toxin solutions that need to be applied at a very precise location and with a very precise quantity.

Similar problems of injecting high viscosity fluids through the skin also occur outside of aesthetic medicine, for example in general surgery.

Some motorized syringes and injection devices already exist on the market. Since the extrusion force needed to expel the fluid through a preferably fine gauge needle is important, many devices require a power cable for powering the actuator in the device. This cable is highly inconvenient for the physician and for the patient.

Some wireless motorized injection devices have been proposed, but usually require a large battery (respectively have a limited autonomy) and a powerful motor or actuator for pushing the piston of the syringe. This results in a heavy, cumbersome and usually expensive injection device that is less convenient to use and manipulate, again leading to lack of control for the physician.

It is an aim of the present invention to mitigate or obviate at least some of the aforementioned disadvantages.

It is another aim of the present invention to provide an injection device that does not require a power cable or a large battery for powering the actuator.

It is another aim of the present invention to provide an injection device that does not require a large size and powerful motor for moving the piston of the syringe.

It is another aim of the present invention to provide an injection device in which the force that is needed from the motor and from the physician during the injection is independent from the viscosity of the fluid that is injected.

### Brief summary of the invention

According to the present invention there is provided an injection device for injecting injection material in medical applications, comprising :
an elastic deformable element such as a spring arranged for exerting a pushing force on a piston of a syringe so as to expel a quantity of injection material out of the syringe;
a stopper;
an electric actuator for moving the stopper from a first position to a second position or from said second position to said first position, so as to release or retain the extension of said elastic deformable element.

The injection device may comprise an elastic deformable element, such as a spring, arranged for exerting a pushing force on a piston of a syringe so as to eject a quantity of injection material out of the syringe;
a stopper for retaining the extension of said elastic deformable element when the stopper is in a first position and for releasing the extension of said elastic deformable element when the stopper is in a second position;
an electric actuator for moving the stopper from its first position to its second position or from its second position to its first position.

This has the advantage that the electric actuator only needs to act on the stopper in order to bring it to stop or release the elastic deformable element that exerts a force on the piston of the syringe. Thus, a small size and low power actuator can be used.

The actuator and stopper thus acts as an escapement to regulate the transfer of mechanical energy from the elastic deformable element to the piston, and to control when the elastic deformable element can release the energy previously stored.

The injection device can comprise a handle connected to said elastic deformable element in order to pre-constrain manually said elastic deformable element. Therefore, the elastic deformable element that pushes the piston of the syringe can be manually pre-constrained in advance by the physician, so that virtually no force needs to be exerted by the physician during the injection. Electric power is thus only used to release the stopper, but not for pushing the piston.

The electric actuator is a linear short-stroke actuator. It is advantageously powered by a battery. Since the actuator needs only little power, a small size battery can be used, and housed in the same housing as the elastic deformable element and the actuator.

The injection device advantageously comprises activation means, such as a button or other haptic means, operable by a user for controlling the electric actuator in order to control ejection of injection material. Therefore, a user merely has to operate this activation means during the injection in order to expel liquid out of the syringe.

The injection device advantageously further comprises a toothed rule with a series of teeth. A stopper may comprise at least one toothed stopper part linearly displaceable relatively to the toothed rule under the action of the elastic deformable element when the stopper is in the second position. The stopper part may comprise a least one tooth engaged with the series of teeth of the toothed rule when the stopper is in the first position.

In one preferred embodiment, the stopper is fixed relative to the housing and the toothed rule is fixed relative to one end of the elastic deformable element and to the piston. In this embodiment, the rule is displaced by the elastic deformable element, causing the piston to move within the syringe when the actuator releases the stopper.

In another embodiment, the stopper is connected with one end of said elastic deformable element and with the piston, and movable with this end relatively to the rule. In this embodiment, the toothed rule is fixed relative to the housing and the stopper moves with one end of the elastic deformable element along this rule.

The stopper may comprise a pivoting lever and two stopper parts with teeth alternatively engageable with the teeth of said toothed rule so as to retain the extension of the elastic deformable element. When one stopper part is blocking the rule, the other one is not. The spacing between the two stopper parts is such that it creates a stepper system. Therefore, a precisely defined fixed volume of injection material is expelled each time the activation means are pressed.

The second position may correspond to an intermediate position of the pivoting lever where none of the stopper parts abuts against the teeth of the toothed rule.

A third position of the stopper may be provided. The displacement of the piston may be released when the stopper is moved from the first to the third position, or alternatively from the third to the first position.

Alternatively, the displacement of the piston may be released when the stopper temporarily leaves the first position and reaches either the second or the third position.

The electric actuator may be a linear actuator acting on said lever, for causing the lever to pivote between the first and the second position.

### Brief Description of the Drawings

The invention will be better understood with the aid of the description of an embodiment, which is given by way of example only, and illustrated by the figures, in which:
Figure 1 provides a perspective view of a complete injection device according to a first or second embodiment of the invention;
Figure 2 provides a perspective view of some components of the injection device of Fig. 1, without the housing;
Figures 3A-3C provide magnified views of one stopper and toothed rule arrangement used in the injection device of the first embodiment of the invention, in three different successive positions.
Figure 4 provides a perspective view of some components of an injection device according to a second embodiment of the invention.
Figures 5-7 provide magnified views of one stopper, toothed rule arrangement and actuator used in the injection device of the second embodiment of the invention, in three different successive positions.

### Detailed Description of possible embodiments of the Invention

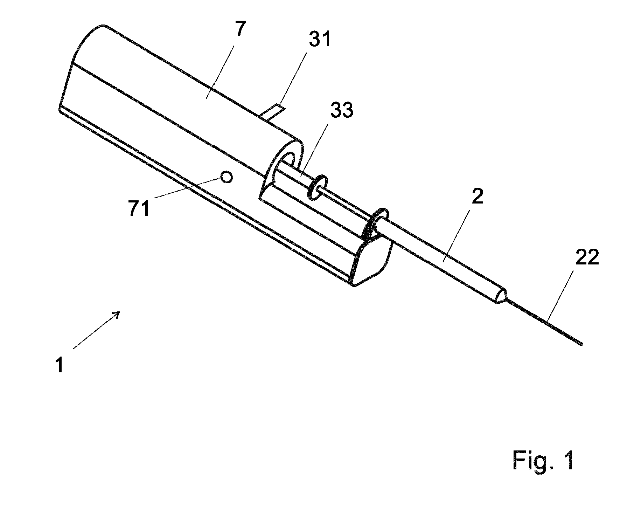

Figure 1 provides a view of a complete injection device 1 according to a first or second embodiment of the invention. In those embodiments, the device 1 is adapted to accommodate and operate a syringe 2 as a consumable that needs to be replaced after each injection or series of injection. The syringe can be conventional and generally comprises a piston 20 that fits in a cylindrical barrel 21.

The piston can be moved by pushing a plunger 33 in the direction of the hypodermic needle 22 at the distal end in order to expel a fluid through the needle. The piston and plunger could be integral in one piece, or in two parts. Please note that the expressions piston and plunger are used interchangeably; the part which is moved within the syringe could be called piston or syringe.

Injection devices where the needle and a separate tank with injection material can be independently replaced could also be considered.

The device 1 further comprises a pen-shaped housing 7 for accommodating the mechanical and electrical parts needed to operate the syringe 1. The size and shape of the housing makes it convenient to use with one hand. A handle 31 is provided on one side or at the proximal end of the housing 7 for retracting the plunger 33 toward the proximal end of the device, and allowing insertion of a new syringe 2 in the device.

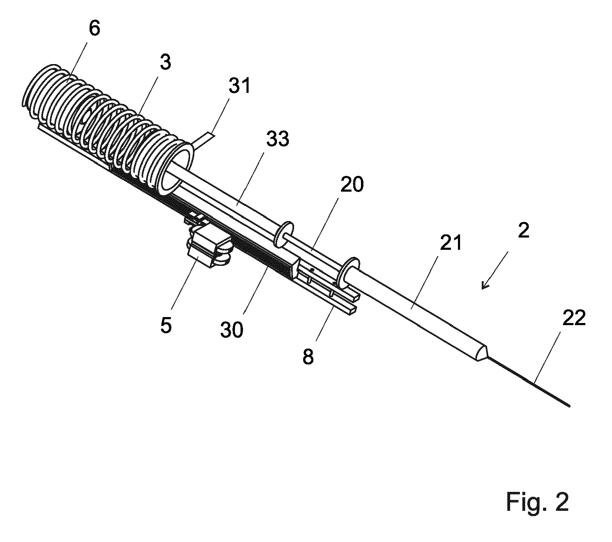

As can be seen on Figure 2 (corresponding to the first embodiment), this displacement results in a preconstraint of a spring 3 against the inner side of the housing 7, in order to store the energy that will be used later to push the piston 20.

The spring could be a coil spring; as illustrated, a coil spring is advantageous since the plunger and/or other components could be accommodated within the spring.

Alternatively, the spring could be one or several blade springs, which occupy a smaller volume than equivalent coil springs.

Instead of a spring, other types of elastic deformable element could be used for storing and releasing the energy, including for example fluids or elastomers.

The element 71 is an activation means, such as a button or a taste that can be manipulated by the physician for controlling the electric actuator 5 and causing the spring to be released and expelling liquid in the syringe 2 through the needle 22. The element 6 is a battery for powering the actuator and the electronics, and that can be housed within the spring 3.

The spring 3 applies a force against the plunger 33 and pushes it in the direction of the piston 20. The distal end of the spring is connected to a toothed rule 30 that moves along the guiding rail 8 when the spring 3 is pre-constrained with the handle 31 and later when it is released. The rail 8 is fixed relative to the housing 7.

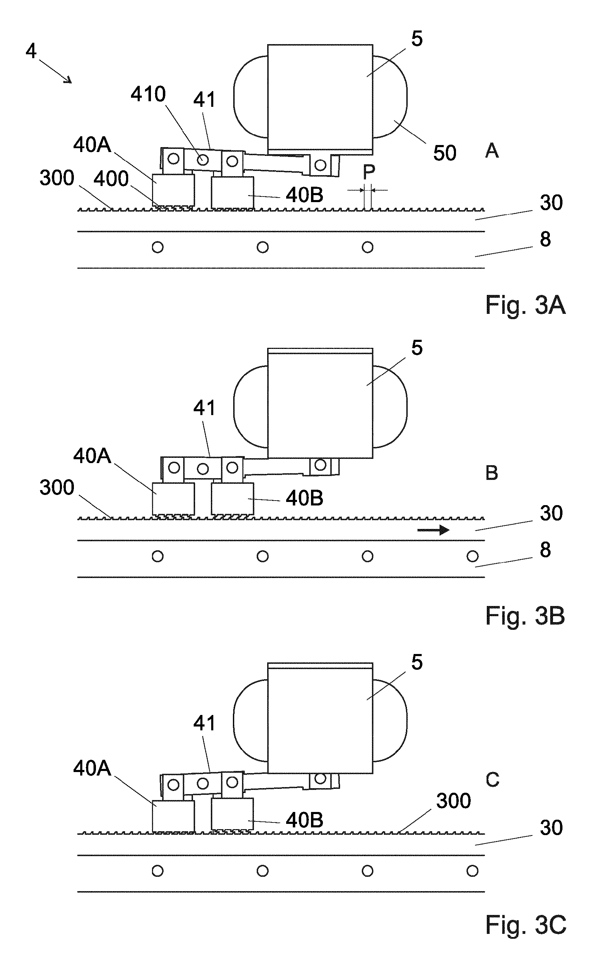

A stopper 4 can be controlled for preventing the rule 30 from moving relative to the stopper when the stopper is in either one of the first or third positions A, C illustrated on Figures 3A and 3B. The displacement of the rule 30 relative to the stopper 4 is released during a short period when the stopper switches from the first position A to the third position C, or from the third position C to the first. Between the first and the third position, the stopper shortly goes through the second position B shown on figure 3B. In this position, the rule 30 moves by one step P toward the distal end.

In the illustrated embodiment, the stopper is fixed relative to the housing and the toothed rule 30 is moved by the spring 3 along the rail 8 in order to push the piston 20. Another embodiment (not shown) could be considered with a fixed toothed rule and a stopper 4 connected to the distal end of the spring 3 and that moves along this rule 30 when released.

The stopper 4 comprises an electric actuator 5, preferably a linear motor with moving magnets that has the advantage of high compacity. A design has been considered with an actuator having a volume of about 1 cm³. A magnet (not shown) is moved linearly by the electromagnetic field induced by the coil 50. A voice coil linear motor could also be considered, although less compact.

The electric actuator 5 controls the position of a pivoting lever 41 that can rotate around the axis 410. Two stopper parts 40A, 40B are mounted on the lever 41; each part has at least one tooth 400 able to cooperate with the teeth 300 of the toothed rule 30 in order to block the displacement of the rule 30 relative to the stopper 4.

In the position illustrated on Figure 3A, the teeth under the surface of the stopper 40B are engaged with the teeth 300 of the rule, which can't move forward.

When the user operates the activation means 71, the actuation means 5 causes the lever 41 to pivote around the axis 410 from the position of Figure 3A to the position of Figure 3B. In this intermediate position, the stopper part 40B is raised. The rule 30 is released and pushed by the spring 3 until it abuts against the teeth 400 of the other stopping part 40A. The piston 20 will be pushed by a length corresponding to the distance P between two adjacent teeth of the rule, for example by half the pitch of the rule 30.

The actuator thus reaches the position of Figure 3C, where the other stopper 40A abuts against the teeth 300 of the rule 30. In both positions of Figures 3A and 3C, conjointly called first position, the displacement of the toothed rule 30 relative to the stopper 4 is blocked by one of the stopper parts 40A or 40B.

Thus, one of the two stopping parts 40A or 40B always prevents the rule 30 from moving, except in the intermediate position of Figure 3B where it is allowed to move by half a pitch. The displacement of the piston 20 at each activation of the means 71 is thus precisely controlled and determined by the distance between the teeth 300 of the rule 30.

In another possible embodiment (not shown), a position of the stopper, for example an intermediate position similar to the one of Figure 3B, could be provided where the rule 30 is free to be displaced continuously under the action of the spring 30. The stopper could be maintained at this position when the activation means 71 are operated and until the next operation of those means. In this case, activation of the means 71 causes a continuous displacement of the piston 20 that will only stop at next activation of the stopper.

It is also possible to imagine an injection device with two different activation means, one for causing a displacement of the rule 30 relative to the stopper 4 by a fixed length P, and another one for raising both stopping parts 40A further, causing a continuous displacement of the rule 30 until next action.

Pre-constraining the spring 3 in order to insert the next syringe 2 is done manually by the physician acting on the handle 31 in order to compress the spring 3 and move the rule 30 (or alternatively the stopper 5) back toward the proximal end. This is done in advance and thus does not require any efforts when the physician actually makes the injection. The handle can preferably only be pulled back when the stopping parts 40A, 40B are in the second position of Figure 3B, or in an even more retracted position so that the teeth 300 are not in contact with the teeth 400 during the retraction. Alternatively, the shape of the teeth 300, 400 could be arranged so as to allow displacement of the rule 30 back toward the distal end, even when the teeth 300 and 400 are mutually engaged. Moreover, it is also possible to consider a motorized displacement of the rule 30 in its backward position.

The injection device 1 may comprise a speed limiting brake (not shown) for preventing a too fast displacement of the moving parts, such as the rule 30 and plunger 33 or alternatively the stopper 4 with the plunger 33. This speed limiting brake may comprise for example a friction element in contact with a moving part.

The injection device 1 may comprise a pressure sensor (not shown) for detecting when the needle has reached a particular body or organ, for example an articular capsule.

The injection device 1 may comprise a retracting element (not shown) for retracting the piston 20 by a fixed, small amount in order to be able to store it with a partly used syringe, without exerting any force against the piston 20 and thus preventing liquid to drop out of the syringe during storage.

The injection device described so far has a pen shape with most components mounted in a line. In another embodiment (not illustrated), a gun shaped injection device could be considered; in this case, the handle of the device could accommodate the battery, the motor and/or the trigger.

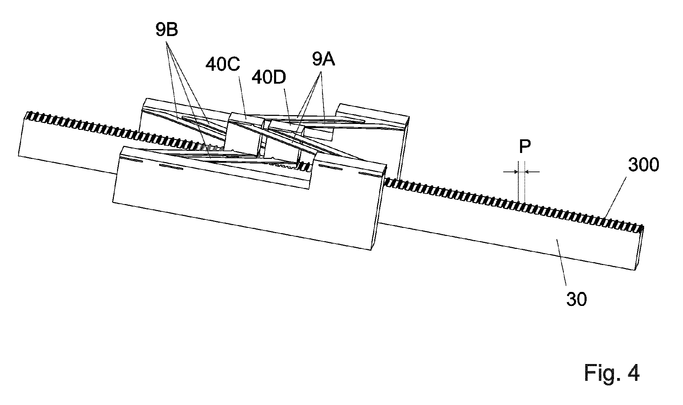

We will now describe a second embodiment of the invention illustrated with Figures 4-7. Parts or components which are not shown on those figures could be identical or similar to the corresponding parts and components described in relation with the first embodiment.

In particular, this second embodiment comprises a toothed rule 30 urged by a spring or other elastic compressible element (not shown) in order to push a piston/plunger and a needle (not shown).

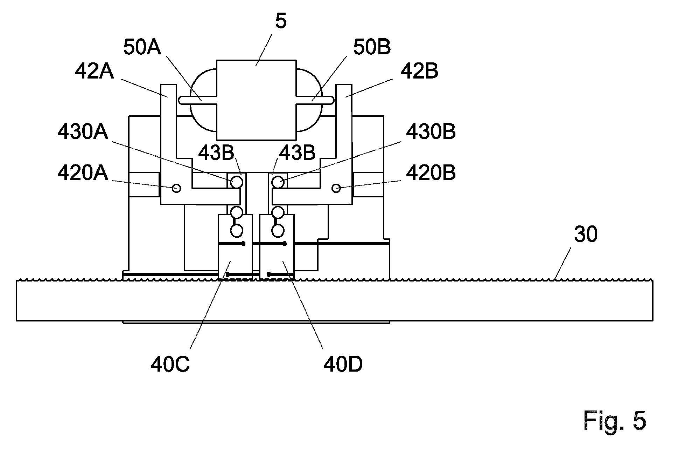

In the first position illustrated on Figure 5, the longitudinal displacement of the toothed rule is blocked by the stopper part 40C, both stoppers parts 40C, 40D being pressed by elastic deformable elements 9A, 9B against the teeth 300 of the rule 30. Each stopper part 40C, 40D has at least one tooth under its lower surface able to cooperate with the teeth 300 of the toothed rule 30 in order to block the displacement of the rule 30 relative to the stopper 4 when the teeth of the stopper and the teeth 300 of the rule 30 are engaged. In the position of Figure 5, only the teeth of the stopper part 40C are in contact with the teeth 300.

In the illustrated example, the elastic deformable elements 9A, 9B consist of blades. Four blades 9A act on the upper parts of both stopper parts 40C, 40D, and four other blades 9B act on the lower parts of both stopper parts 40C, 40D. Therefore, both stopper parts 40C, 40D share the same elastic deformable elements. Those blades guide the displacement of the stopping parts 40A, 40B in a direction orthogonal to the toothed rule 30.

As can be seen on Figures 5 to 7, each of the stopper parts 40C, respectively 40D is connected to one connecting element 43A, respectively 43B so that each stopper part can be retracted against the force exerted by the blades 9A,9B from the teeth 300 when the connecting element is raised or otherwise moved away from the rule. In another not illustrated variant, the connecting element could be integral with the corresponding stopper part.

Each connecting element 43A, 43B is linked to a lever 42A, 42B. In the illustrated example, the connecting elements each comprise two pins 430A, respectively 430B. An extremity of one L-shaped lever 42A respectively 42B is inserted between each pair of pins. The connecting elements 42A, 42B and the associated stopper parts 40C, 40D can thus be retracted away from the teeth 30 when the corresponding lever 42A, 42B is pivoted around an axis 420A, respectively 420B, perpendicular to the rule 30.

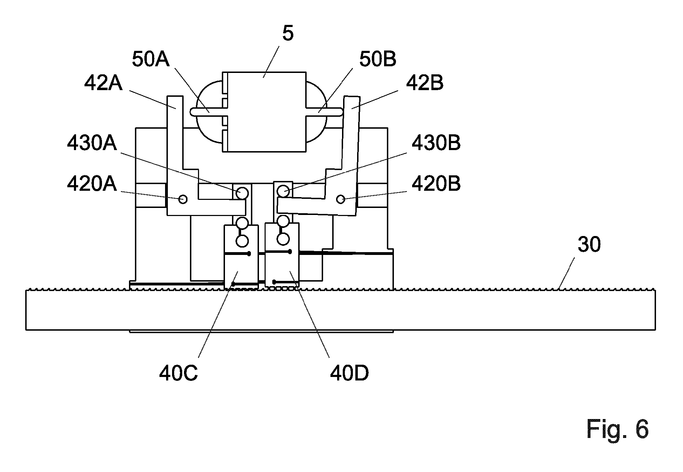

Each of the levers 42A, 42B of the lever arrangement can be pivoted by displacing a hammer 50A, respectively 50B in a direction parallel to the rule 30 by the actuator 5. In one embodiment, the two hammers 50A, 50B consist of the two extremities of a rotor linearly movable by the actuator 5. Therefore, a displacement of the rotor to the left of the figures causes a rotation of the lever 42A and a raise of the connecting element 43A and associated stopper block 40C, which are temporarily moved away from the teeth 300, as shown in the second position illustrated on Figure 6.

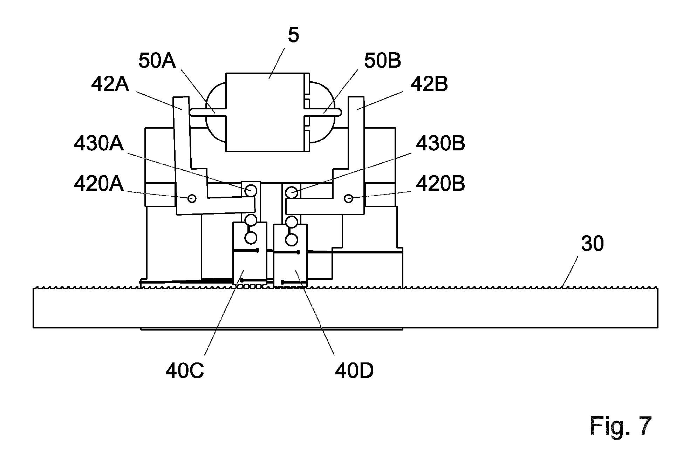

A displacement of the rotor to the right of the figures causes a rotation of the second lever 42B and a raise of the connecting element 43B and associated stopper block 40D, which are temporarily moved away from the teeth 300, as shown in the third position of Figure 7. The stoppers 40C, 40D are then both returned in a position engaged with the teeth 300 by the action of the blades 9A, 9B, in the position illustrated with Figure 5.

When the stopper part 40C that currently blocks the rule 30 is raised, as shown in Figure 6, the rule 30 is released and urged by the spring 3 until it abuts against the teeth of the other stopper part 40D. The piston 20 is pushed by a length corresponding to half the pitch P of the rule 30.

The stopper part 40C is then pushed against the rule (not shown); the rule is blocked by the other stopper part 40D.

Figure 7 shows the next position when this currently blocking stopper part 40D is raised, to release the rule.

In this embodiment, the stopper parts 40C, 40D are never simultaneously raised, thus reducing the risk of a rule being moved by more than half a pitch at a time.

This embodiment also reduced the number and the weight of the moving parts, thus reducing the power requirements. Therefore, a smaller actuator 5 could be used and the autonomy of the battery is increased.

### Reference numbers

- 1: Device
- 2: Syringe
- 20: Piston
- 21: Barrel of the syringe
- 22: Hypodermic needle
- 3: Spring
- 30: Toothed rule
- 300: Teeth of the rule
- 31: Handle
- 33: Plunger
- 4: Stopper
- 40A-D: Stopper parts
- 400: Teeth of the stopper parts
- 41: Pivoting lever
- 410: Axis of the pivoting lever 41
- 42A-B: Pivoting levers
- 420A-B: Axis of the pivoting lever 42
- 43A-B: Connecting element
- 430A-B: Pins
- 5: Electric actuator
- 50A-B: Hammers of the actuator 5
- 6: Battery
- 7: Housing
- 71: Activation means
- 8: Guiding rails
- 9: Spring blade

## Claims

1. An injection device for injecting material in medical applications, comprising :
an elastic deformable element such as a spring (3) arranged for exerting a pushing force on a piston (20) of a syringe (2) so as to expel a quantity of injection material out of the syringe (2);
a stopper (4);
an electric actuator (5) for moving the stopper (4) from a first position (A) to a second position (B) or from said second position (B) to said first position (A), so as to release or retain the extension of said elastic deformable element.

2. The injection device of claim 1, further comprising a toothed rule (30) with a series of teeth (300), said toothed rule cooperating with said stopper (4) and with said piston (20) so as to release or retain the displacement of said piston depending on the position of said stopper (4).

3. The injection device of one of the claims 1 or 2,
wherein said stopper (4) retains the extension of said elastic deformable element (3) when the stopper is in said first position (A) and releases the extension of said elastic deformable element when the stopper is in said second position (B).

4. The injection device of claim 3, wherein said stopper (4) comprises at least one stopper part (40A) linearly displaceable relatively to said toothed rule (30) under the action of said elastic deformable element (3) when said stopper (4) is in said second position,
wherein said at least one stopper part (40A) comprises a least one tooth (300) engaged with the series of teeth (300) of said toothed rule (30) when said stopper (4) is in said first position.

5. The injection device of one of the claims 1 to 4, wherein said actuator is arranged for placing said stopper (4) in either said first position (A), in said second position (B) or in a third position (C),
wherein said stopper (4) retains the extension of said elastic deformable element (3) when the stopper is either in said first position (A) or in said third position (C),
wherein said stopper (4) releases the extension of said elastic deformable element (3) when the stopper moves either from said first position (A) to said third position (C) or from said third position (C) to said first position (A).

6. The injection device of claim 5, wherein said stopper (4) comprises a pivoting lever (41), a first stopper part (40A) and a second stopper part (40B),
said first stopper part (40A) comprising a least one tooth (400) engaged with the series of teeth (300) of said toothed rule (30) when the stopper (4) is in said first position (A),
said second stopper part (40A) comprising a least one tooth (400) engaged with the series of teeth (300) of said toothed rule (30) when the stopper (4) is in said third position (C).

7. The injection device of claim 5, wherein said stopper (4) comprises a pivoting lever (41) and a second stopper part (40B), wherein the first and second stopper parts (40A, 40B) have teeth (400) alternatively engageable with the teeth (300) of said toothed rule (30) so as to retain the extension of said elastic deformable element (30).

8. The injection device of claim 7, wherein both stopper parts (40A, 40B) are mounted on said pivoting lever (41), wherein displacement of said toothed rule (30) relative to said stopper (4) is blocked by either one of said stopper parts when the stopper is in said first position (A), wherein the lever is pivoted when the stopper is in said second position (B) so that the rules abuts alternatively against the first or the second stopper part (40A, 40B).

9. The injection device of claim 8, wherein said actuator (5) is a linear actuator acting on said pivoting lever (41).

10. The injection device of one of the claims 1 to 4, wherein said actuator is arranged for moving said stopper (4) from said first position (A) to either said second position (B) or to a third position (C),
wherein said stopper (4) retains the extension of said elastic deformable element (3) when the stopper is in said first position (A),
wherein said stopper (4) releases the extension of said elastic deformable element (3) when the stopper moves from said first position (A) to said second position (B) or to said third position (C).

11. The injection device of claim 10, wherein said stopper (4) comprises a first stopper part (40C) and a second stopper part (40D),
said first stopper part (40C) comprising a least one tooth (400) engaged with the series of teeth (300) of said toothed rule (30) when the stopper (4) is in said first position (A) or in said third position (C),
said second stopper part (40D) comprising a least one tooth (400) engaged with the series of teeth (300) of said toothed rule (30) when the stopper (4) is in said first position (A) or in said second position (B).

12. The injection device of claim 11, further comprising elastic means (9A, 9b) for urging said first and second stopper parts (40C, 40D) against said toothed rule (30) when said stopper is in said first position.

13. The injection device of claim 12, further comprising a lever arrangement (42A, 42B) actuated by said actuator for alternatively moving said first stopper (40C) or said second stopper (40D) away from said toothed rule (30) so as to temporarily release said toothed rule (30).

14. The injection device of one of the claims 1 to 13, further comprising a handle (31) connected to said elastic deformable element in order to pre-constrain manually said elastic deformable element (3).

15. The injection device of one of the claims 1 to 14, further comprising activation means (71) operable by a user for controlling said electric actuator (5) in order to control ejection of injection material.

16. The injection device of one of the claims 5 to 7, comprising:
a first stopper part (40C);
a second stopper part (40D);
elastic deformable element (9A, 9B) for engaging said first and second stopper parts (40A, 40B) with said toothed rule;
said actuator being arranged for releasing the action of said elastic deformable element (9A, 9B) on either said first or said second stopper part.
